# EUROPEAN PATENT APPLICATION

(11) **EP 3 913 059 A1**
(43) Date of publication of application: **24.11.2021**
(21) Application number: 20175552.7
(22) Date of filing: 19.05.2020
(51) Int. Cl.: C12N 15/82

(54) **NEMATODE RESISTANCE IN PLANTS**

(71) Applicant: Christian-Albrechts-Universität zu Kiel, 24118 Kiel (DE)
(72) Inventor: Jung, Christian, 24118 Kiel (DE); Kumar, Avneesh, 24118 Kiel (DE)
(74) Representative: Taruttis, Stefan Georg

(57) **Abstract**

The invention provides a gene, herein termed *Hs4,* which encodes a protein, also referred to as Hs4, which upon presence in plants confers resistance against plant parasitic nematodes, especially against the beet cyst nematode *Heterodera schachtii* Schmidt. Specifically, the invention provides the use of plants containing the *Hs4* gene for cultivation in the presence of nematodes, in soil of which the presence of nematodes is unknown and in soil that is free from nematodes.

## Description

The present invention relates to a novel resistance gene which upon expression in plants confers resistance against nematodes, especially of the genus *Heterodera,* e.g. against the beet cyst nematode *Heterodera schachtii* Schmidt. For the purpose of the present invention, the novel resistance gene, and respectively the protein encoded by the novel resistance gene, is termed *Hs4.* The invention provides plants which carry the *Hs4* gene, a process for breeding plants using the *Hs4* gene as a marker for identifying plants during the breeding process that carry the *Hs4* gene, a process for generating plants that carry the *Hs4* gene by genetic manipulation to introduce or modify the *Hs4* gene into plants, and to a nucleic acid construct comprising or consisting of the coding sequence of the *Hs4* gene under the control of a promoter that is active in plants, which promoter can be a constitutive promoter and which promoter preferably is a promoter that is induced upon nematode infection, e.g. the *Hs1* beet cyst nematode resistance gene promotor.

### State of the art

Cai et al., Science vol. 275, 832-834 (1997) describe the cloning of a gene encoding a 282 amino acid protein that in sugar beet provides resistance against *Heterodera schachtii.*

Schulte et al., Mol Gen Genomics (2006) describe the generation of a complete physical map of a wild beet translocation site in sugar beet and further mention that the resistance gene described in Cai et al., Science (1997), did not confer complete nematode resistance after transformation into sugar beet.

Heller et al., Theor Appl Genet 92: 991-997 (1996) describe linkage analysis using restriction fragment length polymorphism (RFLP) analysis for localizing nematode resistance genes in sugar beets and for identification of markers linked to the resistance.

EP 3567111 A1 describes three proteins which upon expression in beets confer nematode resistance. The proteins have a length of 1084 amino acids, of 1429 amino acids and of 965 amino acids, respectively.

WO 2014/127835 A1 describes two resistance genes encoding proteins having approx. 929 amino acids for nematode resistance in beets.

Savitsky, H. Can. J. Genet. Cytol. 20:177-186 (1978) describes crossing *Beta vulgaris* and *Beta procumbens* resulting in a translocation of part of chromosome 1 of *Beta procumbens* onto the chromosome 9 of *Beta vulgaris.*

Fauser et al., The Plant Journal 79, 348-359 (2014) describes a method and a plasmid for integration of DNA sections into a plant genome by homologous recombination, making use of CRISPR/Cas methods, especially in a Cas9 expression system plasmid called pChimera.

### Object of the invention

It is an object of the invention to provide an alternative resistance gene that confers resistance against the beet cyst nematode into plants, especially for highly specific identification of the resistance gene in plants, for introducing the resistance gene into plants by genetic manipulation, and to provide a plant breeding process in which plants bearing the resistance gene are identified using the resistance gene as a probe. Preferably, the resistance gene shall provide high resistance against nematodes, especially in *Amaranthaceae.*

### Description of the invention

The invention achieves the object by the features of the claims, and especially provides a gene, herein termed *Hs4,* which encodes a protein, also referred to as Hs4, having an amino acid sequence having a homology of at least 50%, e.g. of at least 60%, of at least 70%, preferably of at least 80%, preferably of at least 85%, more preferably of at least 90% or of at least 95%, most preferably of at least 98% or 99% to MEAVFWIILLNFVIYGAE HLGRVEEIRTLYLIHDRPAWYQFVTSAFCHYNWNHLCNNLF FLYIFGKLVEEEVGGFYLWYYYILTAVGSNLVSWSLLPRSGS SAGASGAVFGLFAISFSVKLLLRDRCPDNKEDWRRFLEVIIL GHFVLQRMMEALHGSNAMVNANGVIDPALVPWVNHIAHLA GAVVGMSLVIIPHDIRRRLSVNNCLPP (SEQ ID N0:1), especially of the amino acid sequence of SEQ ID NO: 1, which upon presence in plants confers resistance against plant parasitic nematodes, especially against the beet cyst nematode *Heterodera schachtii* Schmidt. Specifically, the invention provides the use of plants containing the *Hs4* gene for cultivation in the presence of nematodes, in soil of which the presence of nematodes is unknown and in soil that is free from nematodes, which nematodes preferably are *Heterodera schachtii.*

The protein HS4 can be expressed from a cDNA having a DNA sequence having a homology of at least 80%, preferably of at least 85%, more preferably of at least 90% or of at least 95%, most preferably of at least 98% or 99% to SEQ ID NO: 2, or from a sequence containing exons and introns, e.g. a sequence having a homology of at least 80%, preferably of at least 85%, more preferably of at least 90% or of at least 95%, most preferably of at least 98% or 99% to SEQ ID NO: 3, or having the sequence of SEQ ID NO: 3.

Plants carrying the *Hs4* gene show only little or no yield penalty, i.e. less or no deterioration of yield in production, when cultivated in nematode-free soil, preferably also in nematode-infested soil, compared to wild-type plants without the *Hs4* gene cultivated in nematode-free soil. Especially preferred are plants carrying the *Hs4* gene alone after genetic modification or as part of a small translocation from the *P. procumbens* which can be obtained after crossing different translocation lines or by mutagenesis.

Preferably, a plant containing the *Hs4* gene contains e.g. only a DNA portion containing the *Hs4* gene encoding section and regulatory genetic elements for its expression, which DNA portion consists of at maximum 3 kbp, more preferably at maximum 2.7 kbp, or more preferred of at maximum 1 kbp. The DNA portion containing the *Hs4* gene encoding section and regulatory genetic elements for its expression can be introduced into the plant as a small translocation, e.g. from *P. procumbens,* or by genetic manipulation. Most preferably, the plant in addition to its original DNA only contains the *Hs4* gene comprising or consisting of regulatory genetic elements functionally linked to a coding sequence encoding the *Hs4* gene product, wherein the regulatory genetic elements preferably include a promoter that is induced by presence of nematodes, optionally at least one enhancer in 5' of the coding sequence or in 5' of the promoter. Accordingly, the plant, e.g. in comparison to the genome of the nematode-susceptible cultivar, essentially contains no additional DNA in excess of the *Hs4* gene, which comprises or consists of a coding sequence for the *Hs4* protein and functionally linked regulatory genetic elements. Preferably, the plant containing the *Hs4* gene can contain an insert comprising the *Hs4* gene, which insert has a size of at maximum 100,000 bp, more preferably of at maximum 10,000 bp. The size of the insert can e.g. be determined in comparison to the genome of the plant prior to the integration of the insert comprising the *Hs4* gene.

A preferred promoter that is functionally linked to the nucleic acid sequence encoding the protein having a homology of at least 50%, e.g. of at least 60%, of at least 70%, preferably of at least 80%, preferably of at least 85%, more preferably of at least 90% or of at least 95%, most preferably of at least 98% or 99% to SEQ ID NO: 1, or of SEQ ID NO: 1, is the promoter of SEQ ID NO: 4. As an alternative, the promoter having a homology, e.g. of at least 50%, preferably of at least 80%, more preferably of at least 85%, more preferably of at least 90% or of at least 95%, most preferably of at least 98% or 99% to SEQ ID NO: 4, or comprising or consisting of SEQ ID NO: 4, can be comprised in an expression cassette encoding another protein, e.g. a protein conferring resistance against plant parasitic nematodes, and the expression cassette comprising the promoter can be present in a plant, e.g. by genetic manipulation or by a translocation event.

Preferably, the plant is a member of Amaranthaceae, of Brassicaceae, of Solanaceae, or of Poaceae, e.g. Graminae, especially barley, wheat and rice. Although the invention is not limited to a specific cultivar, the plant preferably is a cultivar of one of the aforementioned genera. Optionally, from the plants according to the invention there are excluded *Patellifolia procumbens,* formerly known as *Beta procumbens,* and/or *Patellifolia patellaris.*

The plant may be a hybrid or a doubled haploid plant, which do not occur in nature.

Analysis of the *Hs4* gene product revealed that upon presence in a plant the *Hs4* gene expresses a protease. Currently it is assumed that the efficacy of the *Hs4* gene to provide resistance against nematodes is based on a detrimental effect of the protease activity on nematodes or on the functioning of the nematode feeding sites. Accordingly, the invention also relates to plants expressing *Hs4,* wherein *Hs4* has protease activity and confers resistance against plant parasitic nematodes.

As the present invention provides the amino acid sequence of the causative gene product for nematode resistance, analysis of plant material for determining the presence of a nucleic acid sequence encoding the causative gene product has become feasible by nucleic acid analytical methods, or by immunological methods that are specific for the *Hs4* gene product. The identification of the *Hs4* gene therefore allows the use of in vitro methods for determining the nematode resistance of a plant, and therefore facilitates the identification of plants that carry the nematode resistance in plant breeding, e.g. reducing the necessity for determining nematode resistance in nematode infection experiments.

The resistance gene *Hs4* can be used for identifying plants that carry a nucleic acid sequence encoding the *Hs4* gene, e.g. using nucleic acid sequences that hybridize to a nucleic acid sequence encoding a protein having a homology of at least 80%, preferably of at least 85%, more preferably of at least 90% or of at least 95%, most preferably of at least 98% or 99% to SEQ ID NO: 1. Processes for identifying the presence of nucleic acid sequences are generally known, e.g. processes using hybridization of nucleic acid probes that are specific, e.g. in PCR, Southern hybridization, or DNA sequencing.

The use of the resistance gene *Hs4* in a process for analysis of plants in respect of the presence of a nucleic acid encoding the *Hs4* gene is useful e.g. in processes for plant breeding for identifying plants that have acquired the *Hs4* gene. Processes for plant breeding can comprise or consist of crossing plants and selecting offspring plants that carry the *Hs4* gene.

Further, the invention relates to a process for genetically manipulating plants by integrating a nucleic acid sequence encoding the *Hs4* gene into the plant genome, and also relates to the genetically manipulated plants, e.g. obtainable by genetic manipulation. According to an embodiment of the invention, genetically manipulated plants contain an insert which essentially consists of the *Hs4* gene including regulatory genetic elements for expression of a protein of SEQ ID NO: 1, with essentially no additional DNA sections. E.g. the insert preferably contains or consists of the coding sequence arranged under the control of a promoter, which preferably is a promoter that is induced by presence of nematodes, e.g. the promoter of SEQ ID NO: 4, and a terminator.

Generally, genetically manipulated plants according to the invention contain an insert comprising the *Hs4* gene which is integrated into the plant genome by genetic manipulation.

Methods for genetic manipulation of plants for integrating DNA into plant genomes are generally known, e.g. using *Agrobacterium* containing a plasmid which comprises a DNA section encoding the *Hs4* gene for transformation of plants using inoculation of the plants with the *Agrobacterium.* Alternatively or additionally, integration of DNA sections into the plant genome can be obtained by genome editing, e.g. CRISPR-Cas based methods.

Methods for genetic manipulation of native protease genes with >80% DNA sequence homology to the *Hs4* gene by genome editing, e.g. CRISPR-Cas based methods. Single or oligonucleotide mutations can be induced in *Hs4* homologs of crop plants, preferably from the plant families Amaranthaceae, Brassicaceae, Poaceae, Solanaceae, whose *Hs4* homologs originally are without a function as a nematode resistance gene, in order to alter their function into a nematode resistance gene, which preferably encodes a protein having a homology of at least 50%, e.g. of at least 60%, of at least 70%, preferably of at least 80%, preferably of at least 85%, more preferably of at least 90% or of at least 95%, most preferably of at least 98% or 99%, or consisting of, SEQ ID NO: 1, which protein preferably is a protease.

The *Hs4* gene of the invention has the advantage of being of plant origin, and to encode a protein that does not significantly impair yield of cultivated plants, e.g. of sugar beets.

The invention is now described in greater detail by way of examples. In the examples, the resistance gene product is also referred to as the *Hs4* gene.

### Example 1: Analysis of the presence of the Hs4 gene in a plant

As an example for analytical methods for determining the presence of specific nucleic acid sequences encoding a protein having a homology of at least 80% to SEQ ID NO: 1, the DNA from leaf samples or root samples from sugar beets was purified. The DNA was analysed by PCR, using the primer of SEQ ID NO: 5 and SEQ ID NO: 6, which between them specifically hybridize to a section of the natural *Hs4* gene, comprising the first exon and part of the 5'UTR of SEQ ID NO: 3. Generally, primer pairs that are specific for amplifying a portion of a known DNA sequence, can be designed using generally available computer programmes on the *Hs4* gene sequence. Amplification conditions were annealing at 58°C, polymerase synthesis at 72°C for 60s, denaturing at 94°C for 30s, for 35 cycles. The amplification product had a size of 194bp.

### Example 2: Generating a plant that contains the Hs4 gene by genetic manipulation

Transfer of the resistance gene *Hs4* into plants can be made using a Ti plasmid and *Agrobacterium tumefaciens* or a Ri plasmid and *Agrobacterium rhizogenes,* wherein the plasmid comprises an expression cassette encoding the *Hs4* gene product.

Alternatively, a nucleic acid sequence encoding a protein of at least 50% homology, preferably of at least 80% homology, more preferably of at least 89% homology to SEQ ID NO: 1 can be integrated into the genome of a plant using a CRISPR-Cas based method.

Another method for introducing the nematode resistance gene *Hs4* into a plant which originally is devoid of the functional *Hs4* gene, especially into sugar beets, more preferably into sugar beet varieties, comprises the steps of crossing the plant with a *Hs4* gene containing plant and from offspring of the crossing selecting those that contain the *Hs4* gene in combination with a desired combination of traits of the plant which originally is devoid of the functional *Hs4* gene. The presence of the *Hs4* gene can be determined by analysing plant material according to Example 1.

### Example 3: Nematode resistance is caused by the Hs4 gene

The sugar beet (*Beta vulgaris*) variety Nemata, which is known as nematode resistant, was genetically manipulated to inactivate the *Hs4* gene specifically. The inactivation of the *Hs4* gene directly resulted in nematode susceptibility of the plants.

In short, target sites for integration were identified manually. As target sites, SEQ ID NO: 7 and SEQ ID NO: 8 were used, which target the exon region of the sugar beet. These target sites were cloned into the pChimera vector (described by Fauser et al., 2014, kindly provided by Prof. Dr. Holger Puchta to the Plant Breeding Institute, CAU Kiel). A single guide RNA having SEQ ID NO: 9 and SEQ ID NO: 10 were cloned into the vector p201G (described by Jacobs et al. 2015, obtainable from Addgene) separately and transformed into *Agrobacterium rhizogenes.* Leaf stalks of the sugar beet were inoculated with the transformed *A. rhizogenes,* and obtained hairy roots were kept on Gamborg B5 medium for two weeks. Hairy roots were tested for mutation in or around target sites by PCR using SEQ ID NO: 11 and SEQ ID NO: 12 as primers and were confirmed by Sanger sequencing. Hairy roots were then inoculated with 250 of J2 *Heterodera schachtii* nematodes, and after four weeks of subsequent cultivation in the dark, female nematodes in hairy roots were counted under the binocular.

The susceptibility of the plants after specific inactivation of the *Hs4* gene demonstrates that this gene confers resistance against nematodes.

Analysis of the originally nematode resistant sugar beet was by PCR, showing the presence of the *Hs4* gene.

## Claims

1. Process for analysis of a plant in respect of resistance against nematodes, **characterized by** analysing nucleic acids of the plant for containing a nucleic acid sequence encoding a protein having a homology of at least 80 % to SEQ ID NO: 1.

2. Process according to claim 1, **characterized in that** the protein of SEQ ID NO: 1 is encoded by a nucleic acid sequence having a homology of at least 80 to SEQ ID NO: 3 or to SEQ ID NO: 2.

3. Resistance gene against plant parasitic nematodes encoding a protein having a homology of at least 80% to SEQ ID NO: 1.

4. Resistance gene according to claim 3, **characterized by** the nucleic acid sequence encoding a protein having a homology of at least 80% to SEQ ID NO: 1 being arranged under the control of a promoter having SEQ ID NO: 4.

5. Resistance gene according to one of claims 3 and 4, **characterized in that** the protein is encoded by a nucleic acid sequence having a homology of at least 80% to SEQ ID NO: 3 or to SEQ ID NO: 2.

6. Resistance gene according to one of claims 3 and 4, **characterized in that** it is comprised in a plasmid vector suitable for gene transfer into plant cells.

7. Resistance gene according to one of claims 3 to 6 for use in protecting plants of the genera of Amaranthaceae, of Brassicaceae, of Poaceae, or of Solanaceae against nematodes.

8. Plant, genetically manipulated to contain a gene conferring resistance against plant parasitic nematodes, **characterized in that** the gene encodes a protein having a homology of at least 80% to SEQ ID NO: 1.

9. Plant according to claim 8, **characterized in that** the gene encoding the protein having a homology of at least 80% to SEQ ID NO: 1 is functionally arranged under the control of a promoter having SEQ ID NO: 4.

10. Plant, genetically manipulated to contain a gene conferring resistance against plant parasitic nematodes, **characterized in that** the gene is under the control of a promoter having a homology of at least 80% to SEQ ID NO: 4.

11. Plant according to one of claims 8 to 10, **characterized in that** the gene conferring resistance against plant parasitic nematodes is comprised in a DNA portion inserted into the plant genome, which DNA portion consists of at maximum 3 kbp.

12. Plant, obtained by a process comprising the step of crossing plants, one of which is a cultivar and the other one of which contains a resistance gene against plant parasitic nematodes encoding a protein having a homology of at least 80% to SEQ ID NO: 1, and the step of selecting offspring plants by identifying offspring plants that contain the resistance gene against plant parasitic nematodes encoding a protein having a homology of at least 80% to SEQ ID NO: 1.

13. Plant according to claim 12, **characterized in that** the offspring plant has essentially all traits of the cultivar and in addition is genetically manipulated such that the resistance gene against plant parasitic nematodes is comprised in a DNA portion inserted into its genome, which DNA portion consists of at maximum 3 kbp.

14. Plant, genetically manipulated to comprise an expression cassette encoding a gene, **characterized in that** the expression cassette contains a promoter having a homology at least 80 % to SEQ ID NO: 4, or the promoter comprising or consisting of SEQ ID NO: 4.


SEQUENCE LISTING

<110> Christian-Albrechts-Universität zu Kiel

<120> Nematode resistance in plants

<130> K1040EP

<160> 12

<170> BiSSAP 1.3.6

<210> 1
<211> 210
<212> PRT
<213> Patellaris procumbens


<220>
<223> nematode resistance gene product

<400> 1
Met Glu Ala Val Phe Trp Ile Ile Leu Leu Asn Phe Val Ile Tyr Gly
1               5                   10                  15
Ala Glu His Leu Gly Arg Val Glu Glu Ile Arg Thr Leu Tyr Leu Ile
            20                  25                  30
His Asp Arg Pro Ala Trp Tyr Gln Phe Val Thr Ser Ala Phe Cys His
        35                  40                  45
Tyr Asn Trp Asn His Leu Cys Asn Asn Leu Phe Phe Leu Tyr Ile Phe
    50                  55                  60
Gly Lys Leu Val Glu Glu Glu Val Gly Gly Phe Tyr Leu Trp Tyr Tyr
65                  70                  75                  80
Tyr Ile Leu Thr Ala Val Gly Ser Asn Leu Val Ser Trp Ser Leu Leu
                85                  90                  95
Pro Arg Ser Gly Ser Ser Ala Gly Ala Ser Gly Ala Val Phe Gly Leu
            100                 105                 110
Phe Ala Ile Ser Phe Ser Val Lys Leu Leu Leu Arg Asp Arg Cys Pro
        115                 120                 125
Asp Asn Lys Glu Asp Trp Arg Arg Phe Leu Glu Val Ile Ile Leu Gly
    130                 135                 140
His Phe Val Leu Gln Arg Met Met Glu Ala Leu His Gly Ser Asn Ala
145                 150                 155                 160
Met Val Asn Ala Asn Gly Val Ile Asp Pro Ala Leu Val Pro Trp Val
                165                 170                 175
Asn His Ile Ala His Leu Ala Gly Ala Val Val Gly Met Ser Leu Val
            180                 185                 190
Ile Ile Pro His Asp Ile Arg Arg Arg Leu Ser Val Asn Asn Cys Leu
        195                 200                 205
Pro Pro
    210

<210> 2
<211> 633
<212> DNA
<213> Artificial Sequence


<220>
<223> cDNA encoding nematode resistance gene product

<400> 2
atggaggcag tattctggat tattcttttg aactttgtta tatacggagc agaacacctt      60

ggcagggttg aagaaatcag aaccctatac ttgatccatg accggcctgc ttggtaccaa 120

tttgtgacct ctgcattttg tcattataat tggaaccatc tttgcaacaa cctattcttt 180

ctgtacattt ttgggaagct cgtcgaagag gaagtgggcg gcttttattt atggtattat 240

tatattctaa cagctgtagg ttcaaacctt gtttcttggt ccctcctccc acgtagtgga 300

agttctgctg gagcttctgg tgctgttttc ggcttgtttg ctataagttt ctcagtgaag 360

ctattactga gagatagatg tccagacaat aaggaagatt ggaggcggtt tcttgaagta 420

attatattag gtcattttgt gttacaaagg atgatggaag cattgcatgg atcaaatgct 480

atggtaaatg caaatggcgt cattgaccca gccttagtac cgtgggtcaa tcatattgcc 540

cacttagccg gtgcagtagt tgggatgagt ttggtaatta tcccccacga catccgtcgt 600

agattatcag tgaataattg tttgccaccc tga 633

<210> 3
<211> 2386
<212> DNA
<213> Artificial Sequence

<220>
<223> coding sequence for nematode resistance gene product including
      untranslated sections

<220>
<221> 5'UTR
<222> 1..179
<223> /note="5´UTR"

<220>
<221> exon
<222> 180..245
<223> /note="exon 1"

<220>
<221> intron
<222> 246..915
<223> /note="intron 1"

<220>
<221> exon
<222> 916..1001
<223> /note="exon 2"

<220>
<221> intron
<222> 1002..1116
<223> /note="intron 2"

<220>
<221> exon
<222> 1117..1157
<223> /note="exon 3"

<220>
<221> intron

<222> 1158..1264
<223> /note="intron 3"

<220>
<221> exon
<222> 1265..1521
<223> /note="exon 4"

<220>
<221> intron
<222> 1522..2017
<223> /note="intron 4"

<220>
<221> exon
<222> 2018..2200
<223> /note="exon 5"

<220>
<221> 3'UTR
<222> 2201..2386
<223> /note="3' UTR"

<400> 3
gtacctagta cctacaaggc catataaaga gacaagcggg taaaccattc ttcattctat 60

actcacattc atattacttt cactaccaaa ggcacagtgt ttactttcac attcatatca 120

tctctacttc cagttggagc ttggaaaacc agttggggaa acgcaaactt aagaagaaga 180

tggaggcagt attctggatt attcttttga actttgttat atacggagca gaacaccttg 240

gcagggtact gaatttatca cttcttttgt ttaagaaact aggtaacagt gtgtcaaagt 300

accatctcaa attctcaatt aaaagtttaa actaatggtt gaagggtcag gttatcatgc 360

ctcttcacac aagacactta taaaggagag gtggatgaga ttcgaactgt gaccttcttg 420

tcacactagc tcttgatatc atgtcaatga accatctcaa ccaaaagctt aaacgtatgg 480

ttgaaggccc aagatatgtt ttatactcta tcagggtgtt tggttcaaat aattggaata 540

aattatatga taatagaaac actatagata taaattcaaa ttccgttgct ttcttataat 600

tgtttggttt accataaagt atggatttaa aagctattac tataggagag gtatggaaca 660

tctataggag tatgagattg aaggggcttt taggggggat aagaatccct tcagtatgcg 720

attccatacc attttacttc aaccaaacaa cattcttcgt taatactagt agccattata 780

aatatagatt tgcatacaag tataacatga tcatgaggag gcggcttttc tttagattgg 840

tcatggcaat tataaggtat agccagtcgc caacttagct attacctctt tatctattga 900

taatgaatct ggcaggttga agaaatcaga accctatact tgatccatga ccggcctgct 960

tggtaccaat ttgtgacctc tgcattttgt cattataatt ggtaagtttc gagactgtaa 1020

tggtcctata tctgtaagac ataagtcatt aatgagtaca gaagatccct ttgaattaat 1080

ttcgtgcttg cgttgcactg ctcgtgttct ccgcaggaac catctttgca acaacctatt 1140

ctttctgtac atttttgtaa ggcaactagc atgtgaaggg cttgaactat actgtgatca 1200

tatcgtcttt gcacttggtt taatttaagg tgtctttcgc ggtttcacca atatatgatt 1260

gcagggaagc tcgtcgaaga ggaagtgggc ggcttttatt tatggtatta ttatattcta 1320

acagctgtag gttcaaacct tgtttcttgg tccctcctcc cacgtagtgg aagttctgct 1380

ggagcttctg gtgctgtttt cggcttgttt gctataagtt tctcagtgaa gctattactg 1440

agagatagat gtccagacaa taaggaagat tggaggcggt ttcttgaagt aattatatta 1500

ggtcattttg tgttacaaag ggtgagaaac tcaatttctt tttatttttc ataagtataa 1560

tttttattat acactcggac ggatcatgag tatagtacac caaagttttt tacttattgt 1620

tcatttgacg acgtcaaaat gagcaataag taaataaaat agtcttgtgt gtctagtata 1680

atagtgctaa gtcaaatgga tttatacgtt gttatcttca acttgttcca agccttatta 1740

gaaaaaaatc agatagattc taatacaatg tgatcctgtc aacaaataaa atctaaagaa 1800

gctcgagttt acataagttc aaataaatgc taatgagtag aggagaacac atttaaaaaa 1860

aatttgaaag ctcttttttc ttcatgttgt ctttgccgaa aagatttcta gtaagttaag 1920

aaaaacagat actaatttat cttttgaaca tgtcttatta catgaaataa aataaggtga 1980

ttacttacaa ttctgccctg tatctttgat aatgtagatg atggaagcat tgcatggatc 2040

aaatgctatg gtaaatgcaa atggcgtcat tgacccagcc ttagtaccgt gggtcaatca 2100

tattgcccac ttagccggtg cagtagttgg gatgagtttg gtaattatcc cccacgacat 2160

ccgtcgtaga ttatcagtga ataattgttt gccaccctga tataaacaaa atgcgggata 2220

aatgtactag tggttaagag ctgcttggtg tattagttga aaagtttctt aaattttgtg 2280

tgttagcgtg tcatgaatgg catcaaagcc catcataacg atttttgttt aatttttgct 2340

atatcttgtt ttcacttttg atgtttccta tacagttttg ggttca 2386

<210> 4
<211> 3083
<212> DNA
<213> Patellifolia procumbens

<220>
<223> inducible promoter

<400> 4
   gagaggagat aagactccac cactggtgcg attcctagct tccaagatgc ttcctatggt 60
   tccacgttct tttatttttg tgtttggtat tgttcgcatc cccctaaaat atattgctat 120
   tgtcacaatt attcgaataa tactattgtg cagtgtgtga cattattcga aataccacag 180
   taaccaatcc cgtttataat actccttccg ttccataata tagtgcttgt ttctcatttt 240
   tcacaaaaat taaggttaga caaaatacca atgattgaag tacttgtgac aacaaaacaa 300
   caaatttaaa gaggtaaagt agggagaaaa aaacaaatat aataatgaca agtacataga 360
   aaaggaatat tttataataa aatgctttgg gggaccattt ctcaagtact tgatgaagat 420
   atgttaggaa tttagtgcct tttaaaaatg gaaacaagaa gaacattttg ggacatccaa 480
   aatggaatac aaacactatt tttatggacg gagggagtag tgctttttta aaaaccaaaa 540
   taagcatgag ttattcttta tatagctttt tatattatcc agttggaata ttcatattaa 600
   agttaagaaa ttctgactat ttttgttgat ttacaagtaa ggaaaaacaa taatcagatt 660
   ttaagtgaaa cttataaatc ttttataatt tatcattaat ttactagcta gtagtattcc 720
   acatctttat agataatttt aatctttatt aaaataatgt ttatattaaa aaaccgagct 780
   agcaattatt tttacataag tactcagtac tcttattatt aactatcaat tgttaataat 840
   tgtcgaaaca tataattaca atttgattag atcactaaaa ctaatactca gtttaaccat 900
   ttctgaatta tctttcattt tttctatata gatattatac ttaattgctt tttatttaat 960
   tattaatttt gtgttgttga gatgcatgga tacatggagt ttacgttgtt ttagttataa 1020
   ttaattgtca gatcaatcaa tatatgttac ttgcatatat attattatgt taaattatat 1080
   tgctataaaa acaacttgca gatatgtcat atcatgtgaa taattcaata cattcgcgac 1140
   gttgaaatga gttttattta tttacttgtt aatttaacta gttagaacta agtaaacatt 1200
   ttgataatga ctaattattt aagtataatt ttctccttgt aaaacaaaaa ttgaatttga 1260
   tcgtttgaat tttatttaag taaaaattat aaaaattgat attaatcaag catatatcga 1320
   gacgaatcta acaagatcgc acatgactat atttatcctt atatataaat cacaaaagaa 1380
   ggtcaacgga aagtgtatga atagtggaaa gtgtaaaaag gttgcaagac atatggaatg 1440
   gaggtagtaa taactaatat atcgattgac gcgtaatttg ccaataaatt ctcacattag 1500
   gtttagggtt taaattgcca ataaaaaagg cgtaaaacgt agccctagat aatttggatt 1560
   actaagcttg atcttctttc ttggtgctta attttttgac actaatccga ttcttagcat 1620
   taagttgaag cacactcttg ataaactatg ttactatgta tcattgtcaa tatgctaaga 1680
   atttgtcttg acctcatcgc tatgtataag cgtataagca tctaatactt tcctaaacta 1740
   gtaaaaacaa atattccatc cgtcccataa tatgagtccc ctttctattt taggagtcaa 1800
   aattttaaaa tttttgacca aatattctta ttactatata taaaaacata ttcatgtggg 1860
   atcttgttag attcgtctta atatgtattt tcataatatc aactttttat atttttttac 1920
   taatacgaaa ttgaagatat acaatgtctt aaagactatg caaaagtaag cagaacctat 1980
   attttgggac ggagggagta ataagtaata ttgattgacg cataatttgt atataaatat 2040
   tttcaaattg atactacttt aaataatata gttaatgctt ataaataagc ctaaagactg 2100
   tgaatagcaa gatcgttaaa aataaaattt gaagccattt ctataaaata tttgtaatgg 2160
   ataatgaaat tggaaatggc atgcttagct tctcgggaat cttataccgc tacatctata 2220
   ataaaaattc ctcataaaat tttgcccatt ttaacacacg aaattcgtcc ttttacgcga 2280
   gccctttcca cacgtcttta aaatttaaaa acctcgtctt tactctcccc acctatatat 2340
   atacacgtcc ccccttctct acttcccatc tcacatacac atacccaatc cacaaacttc 2400
   catcttatcc aactttctct cacctatctc cttcttcaat tttcaaaact caaaagaaaa 2460
   tggtagattt cgattgcaaa acaaaaatgg tacaatcaac accaaacctc acaaaaaaat 2520
   ctccaaaaat cacaaccaaa cgcacaatat caacaccatt aatttcacca gtaccagtaa 2580
   tttccggcga attatctccg gcgtcggaat catcctgttc agcttacgaa tcgtatctca 2640
   aattaccgga gctccgtcaa ctatggagtt caaaagaatt ccccggttgg gataacgaac 2700
   cgataatcaa accggctttg caagcattag agataacatt ccggttcatc tcactcgttt 2760
   tatccgacgc tagaccgtac ataaaccggc gagaatggaa ccggaaatta gagtcgttag 2820
   cgagagatca agtcgaactc atctcagttc tctgcgaaga cgatgagaca cgtggatcag 2880
   ctccgatcgt tgatctgacg tcatcgtatg gtgaggtgat gtcacaaaca ggaagttcag 2940
   cggaggtatg gaagcttgcg aatggagaac atgatactac cgtggtctgt cgtagtagcg 3000
   aatttagtct ccttccgagg ttagccacgt ggcagaagtc ggaggagatt gcttctagaa 3060
   tcttctacgc ggttgaatct gct 3083

<210> 5
<211> 22
<212> DNA
<213> Artificial Sequence

<220>
<223> forward primer

<400> 5
taagaagaag atggaggcag ta 22

<210> 6
<211> 21
<212> DNA
<213> Artificial Sequence

<220>
<223> reverse primer

<400> 6
gaggcatgat aacctgaccc t 21

<210> 7
<211> 20
<212> DNA
<213> Artificial Sequence

<220>
<223> integration target site

<400> 7
agaagatgga ggcagtattc                                                    20


<210> 8
<211> 20
<212> DNA
<213> Artificial Sequence


<220>
<223> integration target site

<400> 8
acggagcaga acaccttggc                                                    20


<210> 9
<211> 107
<212> DNA
<213> Artificial Sequence


<220>
<223> sgRNA target 1

<400> 9
attgagaaga tggaggcagt attcgtttta gagctagaaa tagcaagtta aaataaggct      60

agtccgttat caacttgaaa aagtggcacc gagtcggtgc ttttttt                   107


<210> 10
<211> 107
<212> DNA
<213> Artificial Sequence


<220>
<223> sgRNA target 2

<400> 10
attgacggag cagaacacct tggcgtttta gagctagaaa tagcaagtta aaataaggct      60

agtccgttat caacttgaaa aagtggcacc gagtcggtgc ttttttt                   107


<210> 11
<211> 24
<212> RNA
<213> Artificial Sequence


<220>
<223> forward primer to check editing after CRISPR/Cas

<400> 11
acgcaatgta gcatttctta aaac                                               24

<210> 12
<211> 21
<212> DNA
<213> Artificial Sequence

<220>
<223> reverse primer to check editing after CRISPR/Cas

<400> 12
gaggcatgat aacctgaccc t                                                21